# EUROPEAN PATENT SPECIFICATION

(11) **EP 3 240 505 B1**
(45) Date of publication and mention of the grant of the patent: **05.09.2018**
(21) Application number: 15832957.3
(22) Date of filing: 28.12.2015
(51) Int. Cl.: A61F 2/14

(54) **KERATOPROSTHESIS**
KERATOPROTHESE
KÉRATOPROTHÈSE

(30) Priority: 29.12.2014 US 201414583862
(43) Date of publication of application: 08.11.2017
(73) Proprietor: EyeYon Medical Ltd, 7403649 Nes Ziona (IL)
(72) Inventor: DAPHNA, Ofer, 7680300 Beit Elazari (IL); MARCOVICH, Arie, 7629308 Rehovot (IL); OREN, Guy, 4600840 Herzliya (IL); FERERA, Nahum, 4977415 Petah Tikva (IL)
(74) Representative: Becker Kurig Straus
(86) International application number: PCT/IB2015/060013
(87) International publication number: WO 2016/108175

(56) References cited:
- US-A1- 2010 069 915

## Description

### FIELD OF THE INVENTION

The present invention relates generally to keratoprostheses, that is, devices to replace damaged corneal tissue.

### BACKGROUND OF THE INVENTION

A variety of pathological and accidental causes give rise to damage to the cornea of the eye. Corneal ulceration and resultant scarring are among the most frequent causes of loss of vision worldwide. Ulceration can result from infections, as with Pseudomonas, Staghglococcus, Herpes, and fungi, as well as from chemical and thermal burns, including sometimes after thermokeratoplasty. Ulceration can also occur in association with severe vitamin A and protein deficiency (keratomalacia); in certain dry eye conditions, and in neuroparalytic keratitis; in eyes of patients with autoimmune disease apparently limited to the cornea (Mooren's ulcer), and in association with known systemic autoimmune disorders, like lupus erythematosus and Wegener's granulomatosis, and possible autoimmune diseases like rheumatoid arthritis. Ulceration sometimes progresses to actual perforation with the formation of synechiae between iris and cornea, secondary glaucoma, and even blindness due to death of the optic nerve. Usually, however, after serious insult to the cornea and enzymatic erosion of the stromal matrix, ulceration is arrested, and the cornea, like injured skin, forms scar tissue which scatters light, causing loss of visual acuity. Other mechanical damage can also occur to the cornea, as from scratches or punctures by foreign objects. For these and a variety of other reasons, the corneal portions of eyes must be surgically repaired or replaced.

Corneal transplants have become quite common, particularly in the United States. Unfortunately, donor corneas are very difficult to obtain. A cornea to be donated must be employed, if at all, within a matter of days or weeks from the time of death of the donor.

Various devices have been proposed for solving these problems US 2010/0069915 shows a prior art keratoprosthesis. Glass, polymethyl methacrylate (PMMA), various plastics, polymers, and hydrogels have been tried, with variable success. One known artificial cornea is called the Boston Keratoprosthesis, developed by a team led by Dr. Claes H. Dohlman. It includes a front plate with a stem, which houses the optical portion of the device, a back plate and a titanium locking c-ring. The device has been made from PMMA, but in recent versions the back plate is made from titanium. AlphaCor (Argus Biomedical Pty Ltd, Perth, Australia) is another known keratoprosthetic device. The implant is a 7-mm diameter, one-piece, non-rigid synthetic cornea. It has an opaque, porous, high-water PHEMA (poly[2-hydroxyethyl methacrylate]) outer skirt, with a transparent central optic core of gel PHEMA.

Osteo-odonto-keratoprosthesis (OOKP) is a much different procedure in which a tooth is removed from the patient or a donor, and a layer of tissue from the tooth is fitted with an artificial lens placed in a hole drilled in the tissue lamina. The lamina is grown in the patients' cheek for a period of months and then is implanted upon the eye. The eye must first be prepared by removing the entire inner surface of the eyelids, corneal surface and any scar tissue. This is replaced by transplanting in the eye tissue of the mucosal lining of the cheek. After the tooth bone implant is put in place, the mucosal lining is replaced over the implant.

One known post-operative complication of corneal keratoprosthesis transplant surgery is known as corneal melt. Corneal epithelial defects begin the melting process with failure to re-epithelialize leading to either an infection or a trophic process and subsequent device failure. On the molecular level, immune mediators and collagenases attack the corneal stroma. The two most common causes of corneal melt are herpes simplex virus (HSV) keratitis and retained lenticular material. Another complication is a retroprosthetic membrane (RPM), characterized by an inflammatory/fibrous re-closure of the posterior lamellar opening, usually composed of dense, avascular tissue.

### SUMMARY OF THE INVENTION

Insofar as the terms "invention" and/or "embodiment" are used in the following, and/or features are presented as being optional, this should be interpreted in such a way that the only protection sought is that of the invention as claimed. The present invention seeks to provide a novel and improved keratoprosthesis, as is described further in detail hereinbelow.

There is provided in accordance with an embodiment of the present invention a keratoprosthesis including an anterior collagen layer made of transparent synthetic collagen and a posterior layer made of a material with good or relatively good water-sealing or hydrophobic properties (e.g., silicone layer). The anterior portion of the posterior layer is attached to the posterior portion of the anterior layer. The layers can be the same size or different sizes, that is, the posterior layer can have a bigger radius, same radius or smaller radius compared with the anterior layer.

### BRIEF DESCRIPTION OF THE DRAWINGS

The present invention will be understood and appreciated more fully from the following detailed description taken in conjunction with the drawings in which:
Fig. 1 is a simplified front view illustration of a keratoprosthesis, constructed and operative in accordance with an embodiment of the present invention.
Fig. 2 is a cross sectional view of the keratoprosthesis of Fig. 1, in accordance with one embodiment of the present invention.
Fig. 3 is a cross sectional view of the keratoprosthesis of Fig. 1, in accordance with another embodiment of the present invention.

### DETAILED DESCRIPTION OF EMBODIMENTS

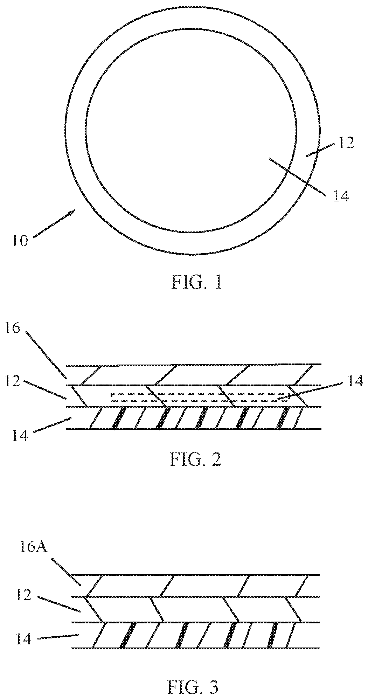

Reference is now made to Fig. 1, which illustrates a keratoprosthesis 10, constructed and operative in accordance with an embodiment of the present invention.

The keratoprosthesis 10 includes a central collagen layer 12 made of transparent synthetic collagen, and shaped as a corneal button. The optical properties of the collagen layer 12, such as but not limited to, convexity, optical power, and refractive properties, are selected for the particular patient. A posterior layer 14 is made of a material with good water-sealing or hydrophobic properties, such as but not limited to, PMMA or silicone and others.

The collagen layer 12, without limitation, has a thickness in the range of 100-550 µm, and a diameter larger than posterior layer 14. With its larger diameter, the collagen layer 12 is in complete 360° peripheral contact with the natural ocular tissue of the recipient. This natural contact should eliminate the problem of corneal melt. However, the invention is not limited to this and the collagen layer 12 may be of the same size or smaller size relative to the posterior layer 14. The posterior layer 14 can be completely posterior to (that is, underneath) the collagen layer 12 (Fig. 2 and 3) or can be positioned in a pocket of the collagen layer 12 (shown in broken lines in Fig. 2). In any case, the anterior surface (that is, the most anterior surface) of the posterior layer 14 is posterior of the anterior surface (that is, the most anterior surface) of the collagen layer 12.

The anterior face of the posterior layer 14 may be bonded to the posterior face of collagen layer 12. Suitable binding agents include for example, but are not limited to, poly-L-lysine and poly-D-lysine or Polymerized N-isopropyl acrylamide (pNIPAM).

The anterior collagen layer serves as a corneal stroma (body) and in a patient with good epithelial reserve, will be covered by the host epithelium. In the embodiment of Fig. 2, an additional anterior layer 16 is disposed over the collagen layer 12. The additional anterior layer 16 may be made of epithelial cells of the recipient, which grows over the collagen layer 12. Alternatively, in the embodiment of Fig. 3, an additional anterior layer 16A is a contact lens, constructed of a suitable lens material, such as but not limited to, PMMA, rigid gas permeable (RGP) polymer, acrylics, silicone and others. The contact lens would be used for those patients with problems of growing epithelial cells, such as with corneal surface problems.

## Claims

1. A device comprising:
a keratoprosthesis (10) comprising an anterior collagen layer (12) made of transparent synthetic collagen; and
a hydrophobic posterior layer (14), a most anterior surface of said posterior layer (14) being posterior of a most anterior surface of said anterior layer (12).

2. The device according to claim 1, further comprising an additional anterior layer (16, 16A) located on the most anterior surface of said collagen layer (12).

3. The device according to claim 2, wherein said additional anterior layer (16) is made of epithelial cells of a recipient of said device.

4. The device according to claim 2, wherein said additional anterior layer (16A) comprises a contact lens.

5. The device according to claim 1, wherein said collagen layer (12) is bonded to said hydrophobic posterior layer (14).

6. The device according to claim 1, wherein said collagen layer (12) has a diameter larger than that of said posterior layer (14).

7. The device according to claim 1, wherein said collagen layer (12) has a diameter equal to that of said posterior layer (14).

8. The device according to claim 1, wherein said collagen layer (12) has a diameter smaller than that of said posterior layer (14).

9. The device according to claim 5, wherein said collagen layer (12) is bonded to said hydrophobic posterior layer (14) with a binding agent.

10. The device according to claim 9, wherein said binding agent comprises poly-L-lysine.

11. The device according to claim 9, wherein said binding agent comprises poly-D-lysine.

12. The device according to claim 9, wherein said binding agent comprises polymerized N-isopropyl acrylamide (pNIPAM).

## Patentansprüche

1. Vorrichtung umfassend:
eine Keratoprothese (10), umfassend eine vordere Kollagenschicht (12), die aus transparentem, synthetischem Kollagen besteht; und
eine hydrophobe hintere Schicht (14), wobei eine vorderste Oberfläche der hinteren Schicht (14) hinter einer vordersten Oberfläche der vorderen Schicht (12) ist.

2. Vorrichtung nach Anspruch 1, ferner umfassend eine zusätzliche vordere Schicht (16, 16A), die sich auf der vordersten Oberfläche der Kollagenschicht (12) befindet.

3. Vorrichtung nach Anspruch 2, wobei die zusätzliche vordere Schicht (16) aus Epithelzellen eines Empfängers der Vorrichtung besteht.

4. Vorrichtung nach Anspruch 2, wobei die zusätzliche vordere Schicht (16A) eine Kontaktlinse umfasst.

5. Vorrichtung nach Anspruch 1, wobei die Kollagenschicht (12) mit der hydrophoben hinteren Schicht (14) verbunden ist.

6. Vorrichtung nach Anspruch 1, wobei die Kollagenschicht (12) einen Durchmesser aufweist, der größer als der Durchmesser der hinteren Schicht (14) ist.

7. Vorrichtung nach Anspruch 1, wobei die Kollagenschicht (12) einen Durchmesser aufweist, der gleich dem Durchmesser der hinteren Schicht (14) ist.

8. Vorrichtung nach Anspruch 1, wobei die Kollagenschicht (12) einen Durchmesser aufweist, der kleiner als der Durchmesser der hinteren Schicht (14) ist.

9. Vorrichtung nach Anspruch 5, wobei die Kollagenschicht (12) mit der hydrophoben hinteren Schicht (14) durch ein Bindemittel verbunden ist.

10. Vorrichtung nach Anspruch 9, wobei das Bindemittel Poly-L-Lysin aufweist.

11. Vorrichtung nach Anspruch 9, wobei das Bindemittel Poly-D-Lysin aufweist.

12. Vorrichtung nach Anspruch 9, wobei das Bindemittel polymerisiertes N-Isopropylacrylamid (pNIPAM) aufweist.

## Revendications

1. Dispositif comprenant :
une keratoprothèse (10) comprenant une couche de collagène antérieure (12) faite de collagène synthétique transparent ; et
une couche hydrophobe postérieure (14), une surface la plus antérieure de ladite couche postérieure (14) étant postérieure d'une surface la plus antérieure de ladite couche antérieure (12).

2. Dispositif selon la revendication 1, comprenant en outre une couche antérieure supplémentaire (16, 16A) située sur la surface la plus antérieure de ladite couche de collagène (12).

3. Dispositif selon la revendication 2, dans lequel ladite couche antérieure supplémentaire (16) est faite de cellules épithéliales d'un bénéficiaire dudit dispositif.

4. Dispositif selon la revendication 2, dans lequel ladite couche antérieure supplémentaire (16A) comprend une lentille de contact.

5. Dispositif selon la revendication 1, dans lequel ladite couche de collagène (12) est collée est à ladite couche hydrophobe postérieure (14).

6. Dispositif selon la revendication 1, dans lequel ladite couche de collagène (12) a un diamètre plus grand que celui de la couche postérieure (14).

7. Dispositif selon la revendication 1, dans lequel ladite couche de collagène (12) a un diamètre égal à celui de la couche postérieure (14).

8. Dispositif selon la revendication 1, dans lequel ladite couche de collagène (12) a un diamètre plus petit que celui de la couche postérieure (14).

9. Dispositif selon la revendication 5, dans lequel ladite couche de collagène (12) est collée est à ladite couche hydrophobe postérieure (14) avec un agent liant.

10. Dispositif selon la revendication 9, dans lequel ledit agent liant comprend de la poly-L-lysine.

11. Dispositif selon la revendication 9, dans lequel ledit agent liant comprend de la poly-D-lysine.

12. Dispositif selon la revendication 9, dans lequel ledit agent liant comprend du N-isopropyle acrylamide polymérisé.
